(19) Europäisches Patentamt
European Patent Office
Office européen des brevets

(11) **EP 3 489 343 A1**

(12) **EUROPEAN PATENT APPLICATION**
published in accordance with Art. 153(4) EPC

(43) Date of publication:
**29.05.2019 Bulletin 2019/22**

(51) Int Cl.:
*C12M 1/00* (2006.01)    *B29C 41/12* (2006.01)
*B32B 3/12* (2006.01)    *B32B 5/32* (2006.01)
*B32B 27/34* (2006.01)    *C08J 9/28* (2006.01)
*C12M 3/00* (2006.01)    *C12M 3/04* (2006.01)
*C12N 1/00* (2006.01)    *C12N 5/00* (2006.01)
*C12N 5/04* (2006.01)    *C12N 5/07* (2010.01)
*C12N 5/10* (2006.01)

(21) Application number: 17834368.7

(22) Date of filing: 25.07.2017

(86) International application number:
**PCT/JP2017/026946**

(87) International publication number:
**WO 2018/021366 (01.02.2018 Gazette 2018/05)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **25.07.2016 JP 2016145822**

(71) Applicant: UBE Industries, Ltd.
**Ube-shi, Yamaguchi 755-8633 (JP)**

(72) Inventors:
• **HAGIHARA, Masahiko**
  **Ube-shi**
  **Yamaguchi 755-8633 (JP)**
• **OHYA, Shyusei**
  **Ube-shi**
  **Yamaguchi 755-8633 (JP)**

(74) Representative: **Plougmann Vingtoft a/s**
**Strandvejen 70**
**2900 Hellerup (DK)**

(54) **CELL PREPARATION METHOD, CELL CULTIVATION DEVICE, AND KIT**

(57)     The present invention relates to a cell preparation method that includes a step in which cells are applied to a polyimide porous film and cultivated, wherein the polyimide porous film is a polyimide porous film with a three-layer structure, having a surface layer A and a surface layer B that have a plurality of holes, and a macrovoid layer that is sandwiched between the surface layer A and the surface layer B, and the polyimide porous film is produced by a method including the following steps: (1) a step in which a poly(amic acid) solution comprising poly(amic acid) and an organic polar solvent is flow cast in a film shape and the result is immersed in or brought into contact with a coagulation medium to create a porous film of poly(amic acid); and (2) a step in which the porous film of poly(amic acid) obtained in step (1) is heat-treated and imidized.

FIG. 1

EP 3 489 343 A1

**Description**

FIELD

The present invention relates to a method for preparing cells, a cell culture apparatus and a kit.

BACKGROUND

A cell culturing method comprising the steps of applying cells to a porous polyimide film and culturing them has been reported (PTL 1).

PRIOR ART DOCUMENTS

PATENT LITERATURE

PTL 1: WO2015/012415

DISCLOSURE OF THE INVENTION

PROBLEMS TO BE SOLVED BY THE INVENTION

PTL 1 reported only that cells were cultured using a colored porous polyimide film obtained by forming a poly(amic acid) solution composition containing a poly(amic acid) solution, which was obtained from a tetracarboxylic acid component and a diamine component, and a colorant precursor, and then applying a heat treatment at 250°C or higher. In this regard, the colorant precursor is a precursor that forms a colored product through the heat treatment at 250°C or higher which partly or entirely carbonizes the precursor. Examples thereof include tar or pitch, such as petroleum tar, petroleum pitch, coal tar, and coal pitch, cokes, a polymer obtained from monomers including acrylonitrile, and a ferrocene compound (ferrocene, and ferrocene derivatives). The porous polyimide film thus produced was brown in color and it was difficult to visually examine the seeding of cells, the engraftment behavior, and the like.

Therefore, an object of the present invention is to simply, efficiently, and stably cultivate cells using a porous polyimide film having better visibility.

MEANS FOR SOLVING THE PROBLEMS

The present inventors have found as a result of intensive studies in view of the aforedescribed problem, that a porous polyimide film having high visibility produced by a method not using a colorant precursor may be used for cell culture, thereby completing the invention.

Namely, the present invention includes the following aspects.

[1] A method for preparing cells, the method comprising the step of:

applying cells to a porous polyimide film and culturing the cells;

wherein the porous polyimide film is a three-layer structure porous polyimide film having a surface layer A and a surface layer B, the surface layers having a plurality of pores, and a macrovoid layer sandwiched between the surface layers A and B;

wherein an average pore diameter of the pores present in the surface layer A is smaller than an average pore diameter of the pores present in the surface layer B;

wherein the macrovoid layer has a partition wall bonded to the surface layers A and B, and a plurality of macrovoids surrounded by the partition wall and the surface layers A and B;

wherein the pores in the surface layers A and B communicate with the macrovoids; and

wherein the polyimide porous is produced by a method comprising the steps of:

(1) casting a poly(amic acid) solution consisting of a poly(amic acid) and an organic polar solvent into a film-like shape, and dipping in or bringing it into contact with a coagulating solvent to prepare a porous film of poly(amic acid); and

(2) imidizing the porous film of the poly(amic acid) obtained in the step (1) by heat treatment.

[2] The method for preparing cells according to [1], wherein the porous polyimide film is produced by a method comprising the steps of:

(1) casting a poly(amic acid) solution consisting of a poly(amic acid) and an organic polar solvent into a film-like shape, and dipping in or bringing it into contact with a coagulating solvent to prepare a porous film of poly(amic acid);

(2) imidizing the porous film of the poly(amic acid) obtained in the step (1) by heat treatment; and

(3) subjecting at least one surface of the porous polyimide film obtained in the step (2) to plasma treatment.

[3] The method for preparing a cell according to [1] or [2], wherein the poly(amic acid) comprises at least one tetracarboxylic dianhydride selected from the group consisting of biphenyltetracarboxylic dianhydride and pyromellitic dianhydride; and at least one diamine selected from the group consisting of benzenediamine, diaminodiphenyl ether and bis(aminophenoxy)phenyl.

[4] The method according to any one of [1] to [3], the method comprising the step of:

seeding cells on the surface of the porous polyimide film.

[5] The method according to any one of [1] to [3], the method comprising the steps of:

placing a cell suspension on the dried surface of the porous polyimide film;

allowing the porous polyimide film to stand, or moving the porous polyimide film to promote efflux of liquid, or stimulating a part of the surface to cause absorption of the cell suspension into the film; and

retaining cells in the cell suspension in the porous polyimide film, and allowing water to flow out.

[6] The method according to any one of [1] to [3], the method comprising the steps of:

wetting one or both sides of the porous polyimide film with a cell culture medium or a sterilized liquid;

loading a cell suspension into the wetted porous polyimide film; and

retaining cells in the cell suspension inside the film, and allowing water to flow out.

[7] The method according to [6], wherein living cells remain within the porous polyimide film, and dead cells flows out with the water.

[8] The method according to [6] or [7], wherein the sterilized liquid is a sterile water or a sterilized buffer solution.

[9] The method according to any one of [1] to [8], the method comprising the step of:

placing a cell culture medium, cells, and one or more of the porous polyimide films in a cell culture vessel, wherein the porous polyimide films are in a suspended state in the cell culture medium.

[10] The method according to [9], characterized in that two or more pieces of the porous polyimide films are used.

[11] The method according to [9] or [10], wherein the cells spontaneously adhere to the porous polyimide film.

[12] The method according to any one of [1] to [8], wherein the porous polyimide film is

i) folded,

ii) wound into a roll-like shape,

iii) connected as sheets or pieces with a filamentous structure, or

iv) bound into a rope-like shape,

and suspended or fixed in a cell culture medium in a cell culture vessel.

[13] The method according to [12], wherein cells spontaneously adhere to the porous polyimide film.

[14] The method according to any one of [1] to [3], the method comprising using two or more porous polyimide films are layered either above and below or left and right in the cell culture medium.

[15] The method according to any one of [1] to [3], wherein two or more of the methods according to any one of [4] to [14] are conducted in combination.

[16] The method according to any one of [1] to [15], wherein cells grow and proliferate on the surface and the inside of a porous polyimide film.

[17] The method according to any one of [1] to [16], wherein the cells are selected from the group consisting of animal cells, insect cells, plant cells, yeasts and bacteria.

[18] The method according to [17], wherein the animal cells are cells derived from an animal belonging to the vertebrate phylum.

[19] The method according to [17], wherein the bacteria are selected from the group consisting of lactic acid bacteria, Escherichia coli, Bacillus subtilis and cyanobacteria.

[20] The method according to any one of [1] to [16], wherein the cells are selected from the group consisting of pluripotent stem cells, tissue stem cells, somatic cells and germ cells.

[21] The method according to any one of [1] to [16], wherein the cells are selected from the group consisting of sarcoma cells, established cells and transformed cells.

[22] A cell culture apparatus for use in a method for preparing cells according to any one of [1] to [21], the apparatus comprising a porous polyimide film.

[23] The cell culture apparatus according to [22], wherein two or more porous polyimide films are layered either above and below or left and right.

[24] A kit for use in a method for preparing cells according to any one of [1] to [21], the kit comprising a porous polyimide film.

EFFECTS OF THE INVENTION

According to the present invention, cells may be simply, efficiently, and stably cultured. In particular, cell seeding, engraftment behavior, *etc.* may be visually confirmed. In addition, the porous polyimide film used is colored only slightly, so it is superior in designability.

BRIEF DESCRIPTION OF DRAWINGS

FIG. 1 represents the time course of the cell number of human dermal fibroblasts cultured using a porous polyimide film.

FIG. 2 represents the time course of the cell number of CHO DP-12 cells cultured using a porous polyimide film.

FIG. 3 represents the time course of the cell number of human mesenchymal stem cells cultured using a porous polyimide film.

FIG. 4 represents the time course of the cell number of human mesenchymal stem cells cultured using a porous polyimide film.

FIG. 5 represents the microscopic observation results with respect to human mesenchymal stem cells cultured on a porous polyimide film.

FIG. 6 represents the light microscope images of human mesenchymal stem cells, which were cultured on a porous polyimide film, and then induced into osteoblasts, on which mineralization was further induced.

FIG. 7 represents the light microscope images of human mesenchymal stem cells, which were cultured on a porous polyimide film, and then induced into adipocytes.

FIG. 8 represents the results of gene analysis after long-term cultivation of human mesenchymal stem cells with a porous polyimide film.

FIG. 9 represents the time course of the cell number of human dermal fibroblasts cultivated for a long period of time using a porous polyimide film.

FIG. 10 represents the amount of fibronectin produced from human dermal fibroblasts cultivated for a long period of time using a porous polyimide film.

DESCRIPTION OF EMBODIMENTS

1. Regarding a porous polyimide film used in the present invention

There is no particular restriction on the average pore diameter of the pores present in the surface layer A (hereinafter also referred to as "A surface" or "mesh surface") of a porous polyimide film used in the present invention, and it is, for example, 0.01 to 50 $\mu$m, 0.01 $\mu$m to 40 $\mu$m, 0.01 $\mu$m to 30 $\mu$m, 0.01 $\mu$m to 20 $\mu$m, or 0.01 $\mu$m to 15 $\mu$m, and is preferably 0.01 $\mu$m to 15 $\mu$m.

There is no particular restriction on the average pore diameter of the pores present in the surface layer B (hereinafter also referred to as "B surface" or "large pore surface") of a porous polyimide film used in the present invention, insofar as it is larger than the average pore diameter of the pores present in the surface layer A. It is, for example, 20 $\mu$m to 100 $\mu$m, 30 $\mu$m to 100 $\mu$m, 40 $\mu$m to 100 $\mu$m, 50 $\mu$m to 100 $\mu$m, or 60 $\mu$m to 100 $\mu$m, and is preferably 20 $\mu$m to 100 $\mu$m.

The average pore diameter of a surface of a porous polyimide film may be found by measuring the pore area with respect to each of 200 or more openings in a scanning electron micrograph of the surface of the porous film, and by calculating the average pore diameter from the average value of the pore areas according to the following Equation (1) assuming that the shape of pores is a perfect circle.

$$\text{Average pore diameter} = 2 \times \sqrt{(S_a / \pi)} \qquad (1)$$

(In the Equation, Sa means the average value of the pore areas.)

There is no particular restriction on the thickness of the surface layer A or B, and it is, for example, 0.01 to 50 μm, and preferably 0.01 to 20 μm.

There is no particular restriction on the average pore diameter of macrovoids in a macrovoid layer of a porous polyimide film in the film planar direction, and it is, for example, 10 to 500 μm, preferably 10 to 100 μm, and more preferably 10 to 80 μm. Further, there is no particular restriction on the thickness of a partition wall in the macrovoid layer, and it is, for example, 0.01 to 50 μm, and preferably 0.01 to 20 μm. At least one partition wall in the macrovoid layer has one or plural pores communicating adjacent macrovoids each other. The average pore diameter of the pores is preferably an average pore diameter of 0.01 to 100 μm, and more preferably 0.01 to 50 μm.

There is no particular restriction on the total film thickness of a porous polyimide film used in the present invention, and it may be 5 μm or more, 10 μm or more, 20 μm or more, or 25 μm or more, and 500 μm or less, 300 μm or less, 100 μm or less, 75 μm or less, or 50 μm or less. It is preferably 5 to 500 μm, and more preferably 25 to 75 μm.

The film thickness of a porous polyimide film used in the present invention can be measured with a contact type thickness measure.

There is no particular restriction on the porosity of a porous polyimide film used in the present invention, and it is, for example, 40% or more and less than 95%.

The porosity of a porous polyimide film used in the present invention may be found from the mass per unit area according to the following Equation (2) by measuring the thickness and the mass of the porous film cut out to a predetermined size.

$$\text{Porosity (\%)} = (1 - w/(S \times d \times D)) \times 100 \qquad (2)$$

(wherein, S is the area of the porous film, d is the total film thickness, w is the measured mass, and D is the density of the polyimide, respectively. The density of the polyimide is assumed to be 1.34 g/cm$^3$.

A porous polyimide film used in the present invention is preferably sterilized. There is no particular restriction on the sterilization treatment, and examples thereof include dry heat sterilization, steam sterilization, sterilization with a disinfectant such as ethanol, and electromagnetic sterilization such as ultraviolet rays and gamma rays.

A porous polyimide film used in the present invention is preferably a three-layer structure porous polyimide film having a surface layer A and a surface layer B, the surface layers having a plurality of pores, and a macrovoid layer sandwiched between the surface layers A and B; wherein an average pore diameter of the pores present in the surface layer A is is 0.01 μm to 15 μm, and an average pore diameter of the pores present in the surface layer is 20 μm to 100 μm; wherein the macrovoid layer has a partition wall bonded to the surface layers A and B, and a plurality of macrovoids surrounded by the partition wall and the surface layers A and B; wherein the partition wall of the macrovoid layer and the surface layers A and B have a thickness of 0.01 to 20 μm, wherein the pores in the surface layers A and B communicate with the macrovoids; and wherein the total film thickness is 5 to 500 μm, the porosity is 40% or more and less than 95%. In this regard, at least one partition wall in the macrovoid layer has one or plural pores, which connect adjacent macrovoids each other, and have an average pore diameter of 0.01 to 100 μm, and preferably 0.01 to 50 μm.

A porous polyimide film used in the present invention is a porous polyimide film containing as a main component a polyimide obtained from a tetracarboxylic dianhydride and a diamine, preferably a porous polyimide film made of a polyimide obtained from a tetracarboxylic dianhydride and a diamine.

The tetracarboxylic dianhydride may be any tetracarboxylic dianhydride, selected as appropriate according to the properties desired. Specific examples of tetracarboxylic dianhydrides include biphenyltetracarboxylic dianhydrides such as pyromellitic dianhydride, 3,3',4,4'-biphenyltetracarboxylic dianhydride (s-BPDA) and 2,3,3',4'-biphenyltetracarboxylic dianhydride (a-BPDA), oxydiphthalic dianhydride, diphenylsulfone-3,4,3',4'-tetracarboxylic dianhydride, bis(3,4-dicarboxyphenyl)sulfide dianhydride, 2,2-bis(3,4-dicarboxyphenyl)-1,1,1,3,3,3-hexafluoropropane dianhydride, 2,3,3',4'-benzophenonetetracarboxylic dianhydride, 3,3',4,4'-benzophenonetetracarboxylic dianhydride, bis(3,4-dicarboxyphenyl)methane dianhydride, 2,2-bis(3,4-dicarboxyphenyl)propane dianhydride, p-phenylenebis(trimellitic acid monoester acid anhydride), p-biphenylenebis(trimellitic acid monoester acid anhydride), m-terphenyl-3,4,3',4'-tetracarboxylic dianhydride, p-terphenyl-3,4,3',4'-tetracarboxylic dianhydride, 1,3-bis(3,4-dicarboxyphenoxy)benzene dianhydride, 1,4-bis(3,4-dicarboxyphenoxy)benzene dianhydride, 1,4-bis(3,4-dicarboxyphenoxy)biphenyl dianhydride, 2,2-bis[(3,4-dicarboxyphenoxy)phenyl]propane dianhydride, 2,3,6,7-naphthalenetetracarboxylic dianhydride, 1,4,5,8-naphthalenetetracarboxylic dianhydride, 4,4'-(2,2-hexafluoroisopropylidene)diphthalic dianhydride, and the like. Also preferably used is an aromatic tetracarboxylic acid such as 2,3,3',4'-diphenylsulfonetetracarboxylic acid. These may be used alone or in appropriate combinations of two or more.

Particularly preferred among these are at least one type of aromatic tetracarboxylic dianhydride selected from the group consisting of biphenyltetracarboxylic dianhydride and pyromellitic dianhydride. As a biphenyltetracarboxylic dianhydride there may be suitably used 3,3',4,4'-biphenyltetracarboxylic dianhydride.

As diamine, any diamine may be used. Specific examples of diamines include the following:

1) Benzenediamines with one benzene nucleus, such as 1,4-diaminobenzene(paraphenylenediamine), 1,3-diaminobenzene, 2,4-diaminotoluene and 2,6-diaminotoluene;

2) diamines with two benzene nuclei, including diaminodiphenyl ethers such as 4,4'-diaminodiphenyl ether and 3,4'-diaminodiphenyl ether, and 4,4'-diaminodiphenylmethane, 3,3'-dimethyl-4,4'-diaminobiphenyl, 2,2'-dimethyl-4,4'-diaminobiphenyl, 2,2'-bis(trifluoromethyl)-4,4'-diaminobiphenyl, 3,3'-dimethyl-4,4'-diaminodiphenylmethane, 3,3'-dicarboxy-4,4'-diaminodiphenylmethane, 3,3',5,5'-tetramethyl-4,4'-diaminodiphenylmethane, bis(4-aminophenyl)sulfide, 4,4'-diaminobenzanilide, 3,3'-dichlorobenzidine, 3,3'-dimethylbenzidine, 2,2'-dimethylbenzidine, 3,3'-dimethoxybenzidine, 2,2'-dimethoxybenzidine, 3,3'-diaminodiphenyl ether, 3,4'-diaminodiphenyl ether, 4,4'-diaminodiphenyl ether, 3,3'-diaminodiphenyl sulfide, 3,4'-diaminodiphenyl sulfide, 4,4'-diaminodiphenyl sulfide, 3,3'-diaminodiphenylsulfone, 3,4'-diaminodiphenylsulfone, 4,4'-diaminodiphenylsulfone, 3,3'-diaminobenzophenone, 3,3'-diamino-4,4'-dichlorobenzophenone, 3,3'-diamino-4,4'-dimethoxybenzophenone, 3,3'-diaminodiphenylmethane, 3,4'-diaminodiphenylmethane, 4,4'-diaminodiphenylmethane, 2,2-bis(3-aminophenyl)propane, 2,2-bis(4-aminophenyl)propane, 2,2-bis(3-aminophenyl)-1,1,1,3,3,3-hexafluoropropane, 2,2-bis(4-aminophenyl)-1,1,1,3,3,3-hexafluoropropane, 3,3'-diaminodiphenyl sulfoxide, 3,4'-diaminodiphenyl sulfoxide and 4,4'-diaminodiphenyl sulfoxide;

3) diamines with three benzene nuclei, including 1,3-bis(3-aminophenyl)benzene, 1,3-bis(4-aminophenyl)benzene, 1,4-bis(3-aminophenyl)benzene, 1,4-bis(4-aminophenyl)benzene, 1,3-bis(4-aminophenoxy)benzene, 1,4-bis(3-aminophenoxy)benzene, 1,4-bis(4-aminophenoxy)benzene, 1,3-bis(3-aminophenoxy)-4-trifluoromethylbenzene, 3,3'-diamino-4-(4-phenyl)phenoxybenzophenone, 3,3'-diamino-4,4'-di(4-phenylphenoxy)benzophenone, 1,3-bis(3-aminophenyl sulfide)benzene, 1,3-bis(4-aminophenyl sulfide)benzene, 1,4-bis(4-aminophenyl sulfide)benzene, 1,3-bis(3-aminophenylsulfone)benzene, 1,3-bis(4-aminophenylsulfone)benzene, 1,4-bis(4-aminophenylsulfone)benzene, 1,3-bis[2-(4-aminophenyl)isopropyl]benzene, 1,4-bis[2-(3-aminophenyl)isopropyl]benzene and 1,4-bis[2-(4-aminophenyl)isopropyl]benzene;

4) diamines with four benzene nuclei, including 3,3'-bis(3-aminophenoxy)biphenyl, 3,3'-bis(4-aminophenoxy)biphenyl, 4,4'-bis(3-aminophenoxy)biphenyl, 4,4'-bis(4-aminophenoxy)biphenyl, bis[3-(3-aminophenoxy)phenyl]ether, bis[3-(4-aminophenoxy)phenyl]ether, bis[4-(3-aminophenoxy)phenyl]ether, bis[4-(4-aminophenoxy)phenyl] ether, bis[3-(3-aminophenoxy)phenyl]ketone, bis[3-(4-aminophenoxy)phenyl]ketone, bis[4-(3-aminophenoxy)phenyl]ketone, bis[4-(4-aminophenoxy)phenyl]ketone, bis[3-(3-aminophenoxy)phenyl] sulfide, bis[3-(4-aminophenoxy)phenyl] sulfide, bis[4-(3-aminophenoxy)phenyl] sulfide, bis[4-(4-aminophenoxy)phenyl] sulfide, bis[3-(3-aminophenoxy)phenyl]sulfone, bis[3-(4-aminophenoxy)phenyl]sulfone, bis[4-(3-aminophenoxy)phenyl]sulfone, bis[4-(4-aminophenoxy)phenyl]sulfone, bis[3-(3-aminophenoxy)phenyl]methane, bis[3-(4-aminophenoxy)phenyl]methane, bis[4-(3-aminophenoxy)phenyl]methane, bis[4-(4-aminophenoxy)phenyl]methane, 2,2-bis[3-(3-aminophenoxy)phenyl]propane, 2,2-bis[3-(4-aminophenoxy)phenyl]propane, 2,2-bis[4-(3-aminophenoxy)phenyl]propane, 2,2-bis[4-(4-aminophenoxy)phenyl]propane, 2,2-bis[3-(3-aminophenoxy)phenyl]-1,1,1,3,3,3-hexafluoropropane, 2,2-bis[3-(4-aminophenoxy)phenyl]-1,1,1,3,3,3-hexafluoropropane, 2,2-bis[4-(3-aminophenoxy)phenyl]-1,1,1,3,3,3-hexafluoropropane and 2,2-bis[4-(4-aminophenoxy)phenyl]-1,1,1,3,3,3-hexafluoropropane.

These may be used alone or in mixtures of two or more. The diamine used may be appropriately selected according to the properties desired.

Preferred among these are aromatic diamine compounds, with 3,3'-diaminodiphenyl ether, 3,4'-diaminodiphenyl ether, 4,4'-diaminodiphenyl ether, paraphenylenediamine, 1,3-bis(3-aminophenyl)benzene, 1,3-bis(4-aminophenyl)benzene, 1,4-bis(3-aminophenyl)benzene, 1,4-bis(4-aminophenyl)benzene, 1,3-bis(4-aminophenoxy)benzene and 1,4-bis(3-aminophenoxy)benzene being preferred for use. Particularly preferred is at least one type of diamine selected from the group consisting of benzenediamines, diaminodiphenyl ethers and bis(aminophenoxy)phenyl.

From the viewpoint of heat resistance and dimensional stability under high temperature, the porous polyimide film is preferably formed from a polyimide obtained by combination of a tetracarboxylic dianhydride and a diamine, having a glass transition temperature of 240°C or higher, or without a distinct transition point at 300°C or higher.

From the viewpoint of heat resistance and dimensional stability under high temperature, the porous polyimide film which may be used for the invention is preferably a porous polyimide film comprising one of the following aromatic polyimides:

(i) An aromatic polyimide comprising at least one tetracarboxylic acid unit selected from the group consisting of biphenyltetracarboxylic acid units and pyromellitic acid units, and an aromatic diamine unit,

(ii) an aromatic polyimide comprising a tetracarboxylic acid unit and at least one type of aromatic diamine unit selected from the group consisting of benzenediamine units, diaminodiphenyl ether units and bis(aminophenoxy)phenyl units,
and/or,
(iii) an aromatic polyimide comprising at least one type of tetracarboxylic acid unit selected from the group consisting of biphenyltetracarboxylic acid units and pyromellitic acid units, and at least one type of aromatic diamine unit selected from the group consisting of benzenediamine units, diaminodiphenyl ether units and bis(aminophenoxy)phenyl units.

2. Regarding the method for producing a porous polyimide film used in the present invention

A porous polyimide film used in the present invention is produced by a method comprising the steps of:

(1) casting a poly(amic acid) solution consisting of a poly(amic acid) and an organic polar solvent into a film-like shape, and dipping in or bringing it into contact with a coagulating solvent to prepare a porous film of poly(amic acid); and
(2) imidizing the porous film of the poly(amic acid) obtained in the step (1) by heat treatment.

A poly(amic acid) is a polyimide precursor constituted with a tetracarboxylic acid unit and a diamine unit, or a partially imidized polyimide precursor therefrom. A poly(amic acid) may be obtained by polymerizing a tetracarboxylic dianhydride, and a diamine. By thermal imidization or chemical imidization of a poly(amic acid) it may be converted to a polyimide through ring closure. A polyimide used in the present invention is preferably produced by thermal imidization. The imidization rate is preferably about 80% or more, more preferably 85% or more, further preferably 90% or more, and still further preferably 95% or more.

As a tetracarboxylic dianhydride and a diamine, those listed in 1. above may be used. A poly(amic acid) used in the method for producing a porous polyimide film used in the present invention is obtained preferably from at least one of tetracarboxylic dianhydride selected from the group consisting of biphenyltetracarboxylic dianhydride, and pyromellitic dianhydride, and at least one of diamine selected from the group consisting of benzenediamine, diaminodiphenyl ether, and bis(aminophenoxy)phenyl.

An arbitrary organic polar solvent may be used as a solvent for polymerizing a poly(amic acid), and examples of a usable organic polar solvent may include *p*-chlorophenol, *o*-chlorophenol, *N*-methyl-2-pyrrolidone (NMP), pyridine, *N,N*-dimethylacetamide (DMAc), *N,N*-dimethylformamide, dimethyl sulfoxide, tetramethylurea, phenol, and cresol. In particular, *N*-methyl-2-pyrrolidone (NMP), *N,N*-dimethylacetamide (DMAc) may be favorably used.

A poly(amic acid) may be produced by an arbitrary method using a tetracarboxylic dianhydride, a diamine, the organic polar solvent, *etc.* For example, a poly(amic acid) solution may be prepared by reacting a tetracarboxylic dianhydride and a diamine quasi equimolarly preferably at a temperature of about 100°C or less, more preferably 80°C or less, further preferably 0 to 60°C, and especially preferably 20 to 60°C, and preferably for about 0.2 hours or more, and more preferably 0.3 to 60 hours.

In preparing the poly(amic acid) solution, an optional molecular weight adjusting component may be added to the reaction solution with a purpose for adjusting the molecular weight.

The intrinsic viscosity number of a poly(amic acid) used in the method for producing a porous polyimide film used in the present invention is preferably 1.0 to 3.0, more preferably 1.3 to 2.8, and especially preferably 1.4 to 2.6.

A poly(amic acid) in which an amic acid is partially imidized may be also used insofar as the present invention is not adversely affected.

The content of a poly(amic acid) in a poly(amic acid) solution is preferably 3 to 60 wt%, more preferably 4 to 40% by mass, further preferably 5 to 20% by mass, and especially preferably 6 to 10% by mass.

A poly(amic acid) solution may be a solution obtained by polymerizing a tetracarboxylic dianhydride and a diamine in the presence of an organic polar solvent, or may be a solution obtained by dissolving a poly(amic acid) in an organic polar solvent.

The solution viscosity of a poly(amic acid) solution is preferably 10 to 10,000 poise (1 to 1000 Pa·s), more preferably 100 to 3,000 poise (10 to 300 Pa·s), further preferably 200 to 2000 poise (20 to 200 Pa·s), and especially preferably 300 to 1000 poise (30 to 100 Pa·s) from the viewpoint of ease of casting and film strength.

(Casting)

In the method for producing a porous polyimide film to be used in the present invention, firstly a poly(amic acid) solution is cast into the film-like shape. There is no particular restriction on the casting method, and for example a poly(amic acid) solution is used as a dope solution and the poly(amic acid) solution is cast onto a glass sheet, a stainless steel sheet, or the like using a T-die or the like into the film-like shape. Alternatively, a poly(amic acid) solution may be intermittently or continuously cast on a movable continuous belt or drum into the film-like shape to produce continuously short pieces or long pieces of a cast sheet. There is no particular restriction on the belt or drum insofar as it is not affected by a poly(amic acid) solution or a coagulating solution, and for example the belt or the drum may be made of a metal such as stainless steel,or a resin such as polytetrafluoroethylene. Further, a poly(amic acid) solution formed into the film-like shape through a T-die may be directly immersed into a coagulating bath. Also, either or both sides of the cast sheet may be brought into contact with a gas containing water vapor or the like (air, inert gas, *etc.*).

(Preparation of porous film of poly(amic acid))

Subsequently, the cast sheet is immersed in or brought into contact with a coagulating solvent containing water as an essential component to precipitate a poly(amic acid) to make it porous thereby forming a porous film. The obtained porous film of a poly(amic acid) may be washed and/or dried according to need.

The coagulating solvent containing water as an essential component is preferably water, or a mixed liquid containing water in a range of 5% by mass or more and less than 100% by mass and an organic polar solvent in a range of more than 0% by mass to not more than 95% by mass. It is more preferable to use a coagulating solvent containing water and an organic polar solvent from the viewpoints of safety from fire, *etc.*, production cost, and securance of the homogeneity of a film to be obtained. Examples of an organic polar solvent which may be contained in a coagulating solvent include an alcohol such as ethanol and methanol, and acetone which are a poor solvent of a poly(amic acid),. Meanwhile, a good solvent of a poly(amic acid) may be added to the extent that the polymer can be precipitated. Specifically, *N*-methyl-2-pyrrolidone (NMP), pyridine, *N,N*-dimethylacetamide (DMAc), and *N,N*-dimethylformamide may be added.

When a coagulating solvent is a mixture of water and an organic polar solvent, the content of water in the coagulating solvent as 100% by mass is preferably 5% by mass or more and less than 100% by mass, more preferably 20% by mass or more and less than 100% by mass, further preferably 30 to 95% by mass, and especially preferably 45 to 90% by mass. The content of an organic polar solvent in the coagulating solvent as 100% by mass is preferably more than 0% by mass and not more than 95% by mass, more preferably more than 0% by mass and not more than 80% by mass, further preferably 5 to 70% by mass, and especially preferably 10 to 55% by mass.

The temperature of a coagulating solvent may be appropriately selected and used according to the purpose, for example, preferably in a range of -30 to 70 °C, more preferably 0 to 60 °C, and further preferably 10 to 50°C.

(Thermal imidization treatment)

Next, the obtained porous film of a poly(amic acid) is thermally treated for imidization to produce a porous polyimide film. Although there is no particular restriction on the thermal imidization treatment, it is preferably performed such that the shrinkage ratio after the treatment each in the longitudinal direction (length direction) and the transverse direction of the film is suppressed to preferably 40% or less, more preferably 30% or less, further preferably 15% or less, still further preferably 8% or less, and especially preferably 5% or less. Although not particularly limited, the thermal treatment may be performed, for example, by fixing a porous film of a poly(amic acid) to a support using a pin, a chuck, pinch rolls, or the like, and heating it in the atmosphere. It is preferable that the reaction conditions should be appropriately selected with respect to the heating temperature in the range of, for example, 280 to 600°C, and preferably 300 to 550°C, and with respect to the heating time in the range of 1 to 120 min, preferably 2 to 120 min, more preferably 3 to 90 min, and further preferably 5 to 30 min.

In the method for producing a porous polyimide film used in the present invention, the rate of temperature increase in a temperature range of 200°C or higher in the thermal imidization treatment is not particularly limited, but it is for example 1°C/min or more, and preferably 5°C/min or more, 10°C/min or more, 15°C/min or more, or 20°C/min or more, more preferably 25°C/min or more, and especially preferably 50°C/min or more. Although it is not particularly necessary to limit the upper limit value of the rate of temperature increase, when the upper limit value of the rate of temperature increase is established, it is, for example, 1 to 500°C/min, preferably 5 to 400°C/min, 5 to 300°C/min, or 5 to 200°C/min, more preferably 50 to 500°C/min, further preferably 50 to 400°C/min, still further preferably 70 to 300°C/min, and especially preferably 120 to 200°C/min.

The porosity, film thickness, average pore diameter in the surface, maximum pore diameter, average pore diameter at the central portion, and the like of a porous polyimide film used in the present invention may be appropriately designed by selecting appropriately the type of polymer used, the polymer concentration, viscosity, organic solution, *etc.*, of a polymer solution, the coagulation conditions (kind of solvent substitution rate adjusting layer, temperature, coagulating solvent, *etc.*), and the like.

In the method for producing a porous polyimide film used in the present invention, the porous polyimide film obtained in the above imidization step may be subjected to a surface treatment, such as a corona discharge treatment, a plasma discharge treatment including a low temperature plasma discharge, and an atmospheric pressure plasma discharge and the like, and a chemical etching, on at least one side of the film according to the purpose. The surface layers A and/or B may be used after machining. These treatments may be carried out according to methods well known to those skilled in the art. It is preferable to apply a plasma discharge treatment to at least one side of a porous polyimide film in order to improve the surface opening diameter, surface opening ratio, and hydrophilicity.

In a preferred embodiment, the method for producing a porous polyimide film used according to the present invention comprises the steps of:

(1) producing a porous film of poly(amic acid) by casting a poly(amic acid) solution composed of 3 to 60% by mass of a poly(amic acid) having an intrinsic viscosity number of 1.0 to 3.0 constituted with a tetracarboxylic acid unit and a diamine unit, and 40 to 97% by mass of an organic polar solvent is cast into a film-like shape, and then immersing in or bringing it into contact with a coagulating solvent containing water as an essential component; and

(2) imidizing the porous film of a poly(amic acid) obtained in the above step by a heat treatment, wherein the shrinkage ratios of the film after the heat treatment in the longitudinal direction and the traverse direction respectively are suppressed to 8% or less, and the rate of temperature increase during the heat treatment in a temperature range of 200°C or higher is 25°C/min or more.

In another preferred embodiment, the method for producing a porous polyimide film used according to the present invention comprises the steps of:

(1) producing a porous film of poly(amic acid) by casting a poly(amic acid) solution composed of 3 to 60% by mass of poly(amic acid) having an intrinsic viscosity number of 1.0 to 3.0 constituted with a tetracarboxylic acid unit and a diamine unit, and 40 to 97% by mass of an organic polar solvent is cast into a film-like shape, and then immersing or bringing it into contact with a coagulating solvent containing water as an essential component;

(2) imidizing the porous film of a poly(amic acid) obtained in the above step by a heat treatment; and

(3) applying a plasma treatment to at least one side of the porous polyimide film obtained in the step (2).

3. Regarding a method for preparing cells according to the present invention

A method for preparing cells according to the present invention comprises application of cells to a porous polyimide film and cultivation thereof. The method according to the present invention is characterized in that it comprises application cells to a porous polyimide film, and cultivation of the cells on the surface of or inside the polyimide film.

(1) Application of cells to porous polyimide film

There are no particular restrictions on the specific steps for application of the cells to the porous polyimide film. It is possible to carry out the steps described throughout the present specification, or to employ any desired method suited for applying cells to a film-like support. Application of cells to the porous polyimide film in the method of the present invention includes, but is not limited to, the following modes:

(A) a mode comprising a step of seeding cells on the surface of the porous polyimide film;
(B) a mode comprising a step of placing a cell suspension on the dried surface of the porous polyimide film, allowing it to stand, or moving the porous polyimide film to promote efflux of the liquid, or stimulating part of the surface to cause absorption of the cell suspension into the film, and retaining the cells in the cell suspension inside the porous polyimide film and allowing the water to flow out; and
(C) a mode comprising a step of wetting one or both sides of the porous polyimide film with a cell culture medium solution or a sterilized liquid, loading a cell suspension into the wetted porous polyimide film, and retaining the cells in the cell suspension inside the porous polyimide film and allowing the water to flow out.

Mode (A) comprises a step of directly seeding cells or a cell mass on the surface of a porous polyimide film. Alternatively, it includes a mode of placing a porous polyimide film in a cell suspension and wetting the cell culture solution from the surface of the film.

Cells seeded on the surface of a porous polyimide film adhere to the porous polyimide film and infiltrate into the interiors of the pores. Preferably, the cells adhere spontaneously to the porous polyimide film without applying any particular exterior physical or chemical force. The cells that have been seeded on the surface of the porous polyimide film can stably grow and proliferate on the surface and/or in the interior of the film. The cells may be in a variety of different forms, depending on the location of the film used for growth and proliferation.

For mode (B), a cell suspension is placed on the dried surface of a porous polyimide film. The porous polyimide film is allowed to stand, or the porous polyimide film is moved to promote efflux of the liquid, or part of the surface is stimulated to cause absorption of the cell suspension into the film, so that the cell suspension permeates into the film. While it is not our intention to be constrained by theory, this is believed to be due to the properties of each surface forms of the porous polyimide film. According to this mode, the cells are absorbed and seeded in the locations of the film where the cell suspension has been loaded.

Alternatively, as according to mode (C), after all or a portion of one or both sides of the porous polyimide film has been wetted with the cell culture solution or sterilized liquid, the cell suspension may be loaded into the wetted porous polyimide film. This will significantly increase the transit rate of the cell suspension.

For example, a method of wetting a portion of the film edges for the main purpose of preventing fly loss of the film (referred to as the "single-point wetting method" hereinbelow) can be used. This method is nearly the same as the dry method (mode (B)) in which the film is not essentially wetted. However, it is possible that cell solution permeation through the film is more rapid at the small wetted portions. A method in which all of one or both sides of the porous polyimide film that have been thoroughly wetted (referred to as "wet film" hereinbelow) is loaded with a cell suspension (referred to as the "wet film method" hereinbelow) can be also used. In this case, the entire porous polyimide film has a greatly increased transit rate for the cell suspension.

According to modes (B) and (C), the cells in the cell suspension are retained in the porous polyimide film, while the water flows out. This allows treatment such as increasing the concentration of cells in the cell suspension and flowing out of unwanted non-cellular components together with the water.

Mode (A) will also be referred to as "natural seeding", and modes (B) and (C) as "suction seeding".

Preferably, but not restrictively, the viable cells are selectively retained in the porous polyimide film. Thus, according to a preferred mode of the invention, the viable cells are retained in the porous polyimide film, and the dead cells preferentially flow out together with the water.

The sterilized liquid used for mode (C) is not particularly restricted, and may be a sterilized buffering solution or sterilized water. A buffering solution may be, for example, (+) or (-) Dulbecco's PBS, or (+) or (-) Hank's Balanced Salt Solution. Examples of buffering solutions are listed in Table 1 below.

[Table 1]

| Component | Concentration (mmol/L) | Concentration (g/L) |
|---|---|---|
| NaCl | 137 | 8.00 |
| KCl | 12.7 | 0.20 |
| $Na_2HPO_4$ | 10 | 1.44 |
| $KH_2PO_4$ | 1.76 | 0.24 |
| pH (-) | 7.4 | 7.4 |

Application of cells to porous polyimide film in the method of the present invention further includes a mode of adding adhesive cells in a floating (suspended) state as a suspension together with a porous polyimide film, to adhere the cells with the film (entangling). For example, for application of the cells to the porous polyimide film in the cell culturing method of the invention, the cell culture medium, the cells and one or more of the porous polyimide films may be placed in the cell culturing vessel. When the cell culture medium is a liquid, the porous polyimide film is in a floating (suspended) state in the cell culture medium. The cells can adhere to the porous polyimide film due to the properties of the porous polyimide film. Thus, even with cells that are not suited for natural suspension culture, the porous polyimide film allows culturing in a floating state in the cell culture medium. The cells preferably spontaneously adhere to the porous polyimide film. Here, "adhere spontaneously" means that the cells are retained on the surface or in the interior of the porous polyimide film without applying any particular exterior physical or chemical force.

Cell culturing can be classified into culturing where the cultured cells are adhesion culture-type cells or suspension culture-type cells, depending on the state in the cell culture. Adhesion culture-type cells are cultured cells that adhere and grow on a culturing vessel, with the medium being exchanged at the time of subculture. Suspension culture-type cells are cultured cells that grow in a suspended state in a medium, and generally the medium is not exchanged at the time of subculture but dilution culture is carried out. Because suspension culture allows culturing in a suspended state, i.e. in a liquid, mass culturing becomes possible, and because it is three-dimensional culturing, unlike with adhering cells that grow only on the culturing vessel surface, the advantage of increased culturable cell count per unit space is afforded.

According to the invention, in conceptual terms, there is provided a method in which it is possible to grow cells in a form similar to suspension culture without being limited to the cell type, so that cells can be conveniently cultured in large amounts. According to the cell culture method of the invention, when the porous polyimide film is used in a state suspended in the cell culture medium, two or more fragments of the porous polyimide film may be used. Since the porous polyimide film is a flexible thin-film, using such fragments that are suspended in the culture solution, for example, allows a porous polyimide film with a large surface area to be added into a fixed volume of cell culture medium. In the case of normal culturing, the container base area constitutes the area limit in which cell culture can be accomplished, but with cell culturing using the porous polyimide film of the invention, all of the large surface area of the previously added porous polyimide film constitutes area in which cell culturing can be accomplished. The porous polyimide film allows the cell culture solution to pass through, allowing supply of nutrients, oxygen and the like even into the folded film, for example.

The sizes and shapes of the porous polyimide film fragments are not particularly restricted. The shapes may be as desired, such as circular, elliptical, quadrilateral, triangular, polygonal or string-like. This includes, for example, quadrilaterals (square, rectangular or the like) and triangular shapes with side lengths of about 0.1 mm to about 20 mm, preferably about 0.2 mm to about 10 mm and more preferably about 1 mm to about 5 mm. Alternatively, for example, they may be circular, with diameters of preferably about 0.1 mm to about 20 mm and more preferably about 0.5 mm to about 10 mm. Dispersing the fragments in the liquid results in a form similar to a suspension culture.

Because the porous polyimide film of the invention is flexible, it can be used with varying shapes. Instead of a flat form, the porous polyimide film can also be used by working into a three-dimensional shape. For example, the porous polyimide film may be: i) folded, ii) wound into a roll, iii) connected as sheets or fragments by a filamentous structure, or iv) bound into a rope, for suspension or fixing in the cell culture medium in the cell culturing vessel. By forming it into shapes such as i) to iv), it is possible to place a large amount of porous polyimide film into a fixed volume of cell culture medium, similar to using fragments. Furthermore, since each fragment can be treated as an aggregate, it is possible to aggregate and move the cell masses, for overall high applicability.

With the same concept as fragment aggregates, two or more porous polyimide films may be used in a layered form either above and below or left and right in the cell culture medium. Layering includes a mode in which portions of the porous polyimide films overlap. Layered culturing allows culturing of cells at high density in a narrow space. It is also possible to further layer a film on a film on which cells are already growing, setting it to create a multilayer of different cell types. This may also be used for drug development, including verification of intercellular interaction in a three-dimensional environment, or in a non-stress cell culture method. The number of layered porous polyimide films is not particularly restricted.

Two or even more forms of the cell culturing method of the invention described above may be used in combination. For example, using any of the methods of modes (A) to (C), first the cells may be applied to the porous polyimide film and then the cell-adhered porous polyimide film may be used for suspension culture. Alternatively, the step of application to the porous polyimide film may be a combination of two or more of the methods of any of modes (A) to (C).

In the method of the invention, preferably the cells grow and proliferate on the surface or in the interior of the porous polyimide film. No reports exist disclosing growth and proliferation of cells inside a three-dimensional structure. By utilization of a porous polyimide film according to the invention it is possible to accomplish continuous three-dimensional culturing of cells. While not restrictive, the method of the invention carries out continuous growth of cells for 2 days or longer, more preferably 4 days or longer and even more preferably 6 days or longer.

(2) Cells used in the present invention

There are no particular restrictions on the type of cells that can be utilized for the method of the invention, and it may be used for growth of any type of cells.

For example, the cells may be selected from the group consisting of animal cells, insect cells, plant cells, yeast cells and bacteria. Animal cells are largely divided into cells from animals belonging to the subphylum Vertebrata, and cells from non-vertebrates (animals other than animals belonging to the subphylum Vertebrata). There are no particular restrictions on the source of the animal cells, for the purpose of the present specification. Preferably, they are cells from an animal belonging to the subphylum Vertebrata. The subphylum Vertebrata includes the superclass Agnatha and the superclass Gnathostomata, the superclass Gnathostomata including the class Mammalia, the class Aves, the class Amphibia and the class Reptilia. Preferably, they are cells from an animal belonging to the class Mammalia, generally known as mammals. Mammals are not particularly restricted but include, preferably, mice, rats, humans, monkeys, pigs, dogs, sheep and goats.

There are also no particular restrictions on sources of plant cells, for the purpose of the present specification. Suitable cells are from plants including bryophytes, pteridophytes and spermatophytes.

Plants from which spermatophyte cells are derived include both monocotyledons and dicotyledons. While not restrictive, monocotyledons include Orchidaceae plants, Poaceae plants (rice, corn, barley, wheat, sorghum and the like) and Cyperaceae plants. Dicotyledons include plants belonging to many subclasses including the subclass Chrysanthemum, the subclass Magnoliidae and the subclass Rosidae.

Algae may be considered cell-derived organisms. These include different groups, from the eubacteria Cyanobacteria (blue-green algae), to eukaryotic monocellular organisms (diatoms, yellow-green algae, dinoflagellates and the like) and multicellular marine algae (red algae, brown algae and green algae).

There are no particular limitations on the types of archaebacteria or bacteria for the purpose of the present specification. Archaebacteria are composed of groups comprising methanogenic bacteria, extreme halophilic bacteria, thermophilic acidophilic bacteria, hyperthermophilic bacteria and the like. Bacteria are selected from the group consisting of, for example, lactic acid bacteria, *E. coli, Bacillus subtilis* and cyanobacteria.

The types of animal cells or plant cells that may be used for the method of the invention are not particularly restricted, but are preferably selected from the group consisting of pluripotent stem cells, tissue stem cells, somatic cells and germ cells.

The term "pluripotent stem cells", for the purpose of the invention, is intended as a comprehensive term for stem cells having the ability to differentiate into cells of a variety of tissues (pluripotent differentiating power). While not restrictive, pluripotent stem cells include embryonic stem cells (ES cells), induced pluripotent stem cells (iPS cells), embryonic germ cells (EG cells) and germ stem cells (GS cells). They are preferably ES cells or iPS cells. Particularly preferred are iPS cells, which are free of ethical problems, for example. The pluripotent stem cells used may be any publicly known ones, and for example, the pluripotent stem cells described in WO2009/123349 (PCT/JP2009/057041) may be used.

The term "tissue stem cells" refers to stem cells that are cells lines capable of differentiation but only to limited specific tissues, though having the ability to differentiate into a variety of cell types (pluripotent differentiating power). For example, hematopoietic stem cells in the bone marrow are the source of blood cells, while neural stem cells differentiate into neurons. Additional types include hepatic stem cells from which the liver is formed and skin stem cells that form skin tissue. Preferably, the tissue stem cells are selected from among mesenchymal stem cells, hepatic stem cells, pancreatic stem cells, neural stem cells, skin stem cells and hematopoietic stem cells.

The term "somatic cells" refers to cells other than germ cells, among the cells composing a multicellular organism. With sexual reproduction, these are not passed on to the next generation. Preferably, the somatic cells are selected from among hepatocytes, pancreatic cells, muscle cells, bone cells, osteoblasts, osteoclasts, chondrocytes, adipocytes, skin cells, fibroblasts, pancreatic cells, renal cells and lung cells, or blood cells such as lymphocytes, erythrocytes, leukocytes, monocytes, macrophages or megakaryocytes.

The term "germ cells" refers to cells having the role of passing on genetic information to the succeeding generation in reproduction. These include, for example, gametes for sexual reproduction, i.e. the ova, egg cells, sperm, sperm cells, and spores for asexual reproduction.

The cells may also be selected from the group consisting of sarcoma cells, established cell lines and transformants. The term "sarcoma" refers to cancer occurring in non-epithelial cell-derived connective tissue cells, such as the bone, cartilage, fat, muscle or blood, and includes soft sarcomas, malignant bone tumors and the like. Sarcoma cells are cells derived from sarcoma. The term "established cell line" refers to cultured cells that are maintained *in vitro* for long periods and reach a stabilized character and can be semi-permanently subcultured. Cell lines derived from various tissues of various species including humans exist, such as PC 12 cells (from rat adrenal medulla), CHO cells (from Chinese hamster ovary), HEK293 cells (from human embryonic kidney), HL-60 cells (from human leukocytes), HeLa cells (from human cervical cancer), Vero cells (from African green monkey kidney epithelial cells), MDCK cells (from canine kidney renal tubular epithelial cells) and HepG2 cells (from human hepatic carcinoma). The term "transformants" refers to cells with an altered genetic nature by extracellularly introduced nucleic acid (DNA and the like). Suitable methods are known for transformation of animal cells, plant cells and bacteria.

(3) Cell culture system and cell culture conditions

In the method of the present invention, the cell culture system and the culture conditions may be appropriately determined according to the type of cells and the like. A culture method suitable for each cell type, such as animal cells, plant cells, and bacterial cells has been known, and those skilled in the art can cultivate cells with a porous polyimide film using an appropriate known method. The cell culture medium may also be appropriately prepared according to the type of cells.

A cell culture method, and a cell culture medium for animal cells are described in, for example, the cell culture medium catalog of Lonza. A cell culture method and a cell culture medium for plant cells are described in, for example, the Plant Tissue Culture Medium Series catalog of Wako Pure Chemical Industries, Ltd. A cell culture method, and a cell culture medium for bacterial cells are described, for example, in the general purpose bacterial medium catalog of Becton, Dickinson and Company. The cell culture medium used in the method of the present invention may be in any form such as liquid medium, semi-solid medium, and solid medium. Further, a liquid medium in the form of droplets may be sprayed into a cell culture container, such that the medium is brought into contact with a porous polyimide film supporting cells.

With respect to cell culture using a porous polyimide film, another suspension culture carrier, such as a micro carrier and a cellulose sponge, may coexist.

In a method of the present invention, there is no particular restriction on the shape, the scale, and the like of the system used for cultivation, and any of a petri dish, a flask, a plastic bag, a test tube, and a large tank for cell culture may be appropriately used. Examples thereof include BD Falcon cell culture dishes, and Nunc Cell Factory System manufactured by Thermo Fisher Scientific Inc. By using a porous polyimide film in the present invention, it became possible to carry out cultivation in a state similar to a suspension culture in a device for suspension culture also for cells to which a suspension culture was not applicable by nature. As a device for suspension culture, for example, a spinner flask manufactured by Corning Inc., or a rotating incubator and the like may be used. Also a hollow fiber culture, such as FiberCell (registered trademark) System of Veritas Corp, may be used to provide an environment where the same function can be realized.

Cultivation in the method of the present invention may be carried out in a mode where a porous polyimide film sheet is exposed to the air using a device for continuous circulation in which a medium is added continuously onto a porous polyimide film and then recovered, or an open-type device.

In the present invention, the cell culture may be carried out in a system in which a cell culture medium is continuously or intermittently supplied from a cell culture medium supplying means installed outside a cell culture container into the cell culture container. In this regard, it may be the system in which the cell culture medium circulates between the cell culture medium supplying means and the cell culture container.

In a case where the cell culture is carried out in a system in which a cell culture medium is continuously or intermittently supplied from a cell culture medium supplying means installed outside a cell culture container into the cell culture container, the system may be a cell culture apparatus comprising a culture unit constituted with a cell culture container, and a medium supply unit constituted with a cell culture medium supplying means, and in the cell culture apparatus:

the culture unit may be a culture unit which accommodates one or plural porous polyimide films for carrying cells, and is equipped with a medium supply port and a medium discharge port,

the medium supply unit may be a medium supply unit which is provided with a medium storage container, a medium supply line, and a liquid feed pump for continuously or intermittently feeding the medium via the medium supply line, wherein the first terminal of the medium supply line is in contact with the medium in the medium storage container, and the second terminal of the medium supply line is connected to the inside of the culture unit via the medium supply port of the culture unit.

Further, regarding the cell culture apparatus, the culture unit may be a culture unit which is not provided with an air supply port, an air discharge port, or an oxygen exchange film, or may be a culture unit which is provided with an air supply port and an air discharge port, or with an oxygen exchange film. Even when a culturing unit is not provided with an air supply port and an air discharge port, nor with an oxygen exchange film, oxygen and the like necessary for cell culture are sufficiently supplied to cells through the medium. Furthermore, in the cell culture apparatus, the culture unit may be further provided with a medium discharge line, wherein the first terminal of the medium discharge line is connected to the medium storage container, and the second terminal of the culture medium discharge line is connected to the inside of the culture unit via the medium discharge port of the culture unit, so that the medium is able to circulate between the medium supply unit and the culture unit.

4. Regarding a cell culture apparatus of the present invention

The present invention also relates to a cell culture apparatus, which comprises a porous polyimide film, and is used in the preparation method of the present invention. In the cell culture apparatus of the present invention, the porous polyimide film may be used in a fixed state, or in a state suspended in the cell culture medium. In the cell culture apparatus, two or more porous polyimide films may be layered either above and below or left and right.

5. Kit of the present invention

The present invention also relates to a kit for use in the method for preparing cells, the kit comprising a porous polyimide film.

The kit of the invention may comprise constituent elements necessary for cell culturing in addition to the porous polyimide film, as appropriate. This comprises, for example, the cells applied to the porous polyimide film, the cell culture medium, the cell culturing apparatus and the instruction manual for the kit.

While not restrictive, one mode includes a package containing either one or a plurality of sterilized porous polyimide films stored in a transparent pouch, in a form allowing their use for cell culturing, or a kit having sterile liquid encapsulated together with a porous polyimide film in the same pouch, in the form of an integrated film/liquid allowing efficient suction seeding.

EXAMPLES

Although the present invention will be described below in more detail with reference to the following Examples, it goes without saying that the present invention is not limited in any means by the Examples.

Example 1

Time course of the cell number of human dermal fibroblasts cultured using a porous polyimide film

1. Preparation of porous polyimide films 1 and 3

(1) Preparation of a poly(amic acid) solution composition A

Into a 500 mL separable flask, 3,3',4,4'-biphenyltetracarboxylic dianhydride (s-BPDA) as an acid anhydride, and 4,4'-diaminodiphenyl ether as a diamine were weighed out and charged such that the molar ratio of acid anhydride/diamine became 0.994 and the polymer concentration became 8% by mass using *N*-methyl-2-pyrrolidone (NMP) as a solvent. Then the flask was closed with a separable cover equipped with a stirring impeller, a nitrogen feed tube, and an exhaust tube, and a stirring operation was continued for 30 hours. After completion of the stirring, the dope in the flask was filtrated with a pressure filter (Filter paper No. 60 for viscous liquid, produced by Advantec Toyo Kaisha, Ltd.) to yield a poly(amic acid) solution composition A. The solution composition A was a viscous liquid with a viscosity of 320 poise. The intrinsic viscosity number was 1.6.

(2) Preparation of porous polyimide films 1 and 3

The poly(amic acid) solution composition A was coated on a substrate, which is a square of side 20 cm, and made of stainless steel having a mirror polished surface, by casting uniformly using a desktop automatic coater at room temperature to a thickness in a range of about 100 to 300 $\mu$m. After being left standing in the air at a temperature of 23°C and a humidity of 40% for 90 sec, the entire substrate was dipped into a coagulating bath (80 parts by mass of water, and 20 parts by mass of NMP, room temperature). After dipping it was left to stand there still for 8 min, so as to deposit a poly(amic acid) film on the substrate. Thereafter, the substrate was taken out from the bath, and the poly(amic acid) film deposited on the substrate was peeled off, and then immersed in pure water for 3 min to obtain a poly(amic acid) film. The poly(amic acid) film was dried in the air at a temperature of 23°C and a humidity of 40%, and then stuck to a 10 cm-square pin tenter and the four sides were fixed. The fixed film was placed in an electric furnace for a heat treatment with such a temperature profile, that the temperature was raised to 150°C at a rate of temperature increase of about 10°C/min, then further raised to the maximum temperature of 340°C, and kept there for 3 min. Thus, a porous polyimide film 1 (25 $\mu$m), and a porous polyimide film 3 (48 $\mu$m) having different thicknesses were prepared. The porous polyimide films 1 and 3 were hereinafter also referred to as "film 1" and "film 3", respectively. Both of them were a three-layer structure porous polyimide film having a surface layer A and a surface layer B, the surface layers having a plurality of pores, and a macrovoid layer sandwiched between the surface layer A and the surface layer B.

With respect to the film 1, the average pore diameter of the pores present in the surface layer A was 21 $\mu$m, the average pore diameter of the pores present in the surface layer B was 32 $\mu$m, and the porosity was 73%.

With respect to the film 3, the average pore diameter of the pores present in the surface layer A was 18 $\mu$m, the average pore diameter of the pores present in the surface layer B was 28 $\mu$m, and the porosity was 76%.

2. Preparation of porous polyimide films 2 and 4

(1) Preparation of a poly(amic acid) solution composition B

Into a 500 mL separable flask, 3,3',4,4'-biphenyltetracarboxylic dianhydride (s-BPDA) as an acid anhydride, and 4,4'-diaminodiphenyl ether as a diamine were weighed out and charged such that the molar ratio of acid anhydride/diamine became 0.996 and the polymer concentration became 8% by mass using *N*-methyl-2-pyrrolidone (NMP) as a solvent. Then the flask was closed with a separable cover equipped with a stirring impeller, a nitrogen feed tube, and an exhaust tube, and a stirring operation was continued for 30 hours. After completion of the stirring, the dope in the flask was filtrated with a pressure filter (Filter paper No. 60 for viscous liquid, produced by Advantec Toyo Kaisha, Ltd.) to yield a poly(amic acid) solution composition. The solution composition was a viscous liquid with a viscosity of 452 poise. The intrinsic viscosity number was 2.4.

(2) Preparation of porous polyimide films 2 and 4

The poly(amic acid) solution composition B was coated on a substrate, which is a square of side 20 cm, and made of stainless steel having a mirror polished surface, by casting uniformly using a desktop automatic coater at room temperature to a thickness of about 100 to 200 $\mu$m. After being left standing in the air at a temperature of 23°C and a humidity of 40% for 90 sec, the entire substrate was dipped into a coagulating bath (80 parts by mass of water, and 20 parts by mass of NMP, room temperature). After dipping it was left to stand there still for 8 min, so as to deposit a poly(amic acid) film on the substrate. Thereafter, the substrate was taken out from the bath, and the poly(amic acid) film deposited on the substrate was peeled off, and then immersed in pure water for 3 min to obtain a poly(amic acid) film. The poly(amic acid) film was dried in the air at a temperature of 23°C and a humidity of 40%, and then stuck to a 10 cm-square pin tenter and the four sides were fixed. The fixed film was placed in an electric furnace for a heat treatment with such a temperature profile, that the temperature was raised to 150°C at a rate of temperature increase of about 10°C/min, then further raised to the maximum temperature of 340°C, and kept there for 3 min, to yield a polyimide porous membrane.

Thereafter, a normal pressure plasma treatment was applied to one side of the obtained porous polyimide film for 60 sec to yield polyimide porous films 2 and 4, which are hereinafter also referred to as "film 2" and "film 4", respectively. Both of the films were a three-layer structure porous polyimide film having a surface layer A and a surface layer B, the surface layers having a plurality of pores, and a macrovoid layer sandwiched between the surface layer A and the surface layer B.

With respect to the film 2, the average pore diameter of the pores present in the surface layer A was 9 $\mu$m, the average pore diameter of the pores present in the surface layer B was 33 $\mu$m, the thickness was 25 $\mu$m, and the porosity was 74%.

With respect to the film 4, the average pore diameter of the pores present in the surface layer A was 8 $\mu$m, the average pore diameter of the pores present in the surface layer B was 35 $\mu$m, the thickness was 48 $\mu$m, and the porosity was 78.9%.

The features of porous polyimide films with respect to films 1 to 4 are presented in the following table.

[Table 2]

|  | Plasma irradiation treatment | Film thickness ($\mu$m) | Porosity (%) | Average pore diameter of pores present in surface layer A ($\mu$m) | Average pore diameter of pores present in surface layer B ($\mu$m) | Appearance |
|---|---|---|---|---|---|---|
| Film 1 | No | 25 | 73 | 21 | 32 | Yellowish-white |
| Film 2 | Yes | 25 | 74 | 9 | 33 | Yellowish-white |
| Film 3 | No | 48 | 76 | 18 | 28 | Yellowish-white |
| Film 4 | Yes | 48 | 79 | 8 | 35 | Yellowish-white |

3. Culture of human dermal fibroblasts using films 1 to 4

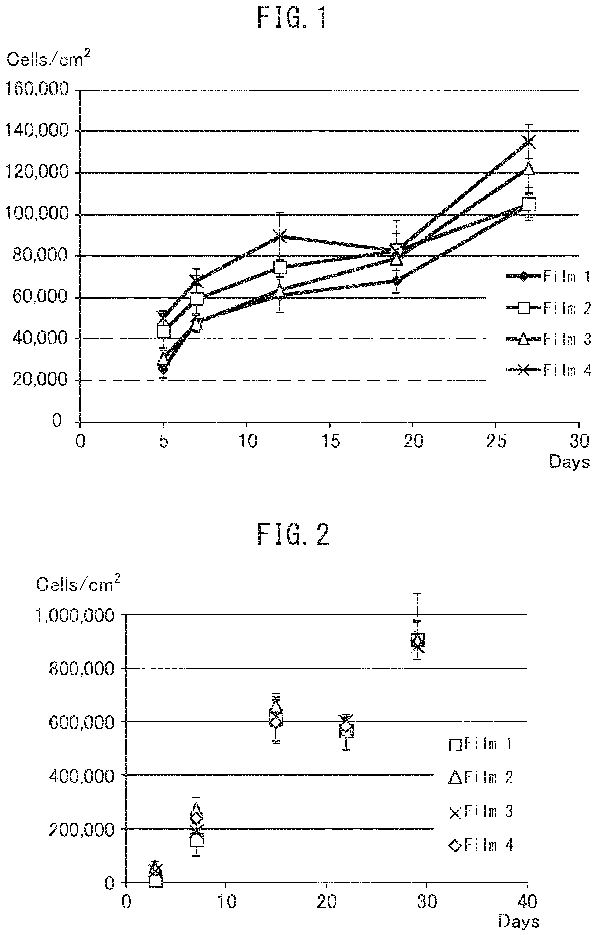

Into a sterilized square container with a size of 2 cm × 2 cm (Thermo Fisher Scientific Inc., cat. 103), 1 mL of a medium for cultivating human fibroblasts (LONZA, CC-3132) was added, and a sterilized 1.4 cm-square films 1 to 4 were placed at rest with the mesh-structured surface A up, or the large pore-structured surface B up. The human dermal fibroblasts (CC-2511 from LONZA) in a number of $4 \times 10^4$ were seeded per sheet, and incubated continuously in a $CO_2$ incubator. The medium (1 mL) was exchanged twice a week. On day 5, 7, 12, 19, and 27 from the initiation of incubation, the cell number was counted using a Cell Counting Kit-8 (manufactured by Dojindo Laboratories, hereinafter referred to as "CCK 8"), and the cell growth behavior was observed. The results are depicted in FIG. 1. It was confirmed that substantially the same number of cells could be stably cultured in the films 1 to 4.

Example 2

Time course of the cell number of CHO DP-12 cells cultured using a porous polyimide film

Into a sterilized square container with a size of 2 cm × 2 cm (Thermo Fisher Scientific Inc., cat. 103), 0.5 mL of a cell culture medium (IMDM 098-06465, Wako Pure Chemical Industries, Ltd.) was added, and the sterilized 1.4 cm-square films 1 to 4 (those prepared in Example 1) were placed with the mesh-structured surface A up, and the anti-human IL-8 antibody-producing CHO DP-12 cells (ATCC CRL-12445) in a number of $4 \times 10^4$ were seeded per sheet. Incubation was continued in a $CO_2$ incubator, and the medium was exchanged periodically twice a week. On day 3, 7, 15, 22, and 29 from the initiation of incubation, the cell number was counted using a CCK 8, and the cell growth behavior was observed. The results are depicted in FIG. 2. Stable cell growth was observed. It was confirmed that substantially the same number of cells could be stably cultured in the films 1 to 4.

Example 3

Time course of human mesenchymal stem cells cultured using a porous polyimide film

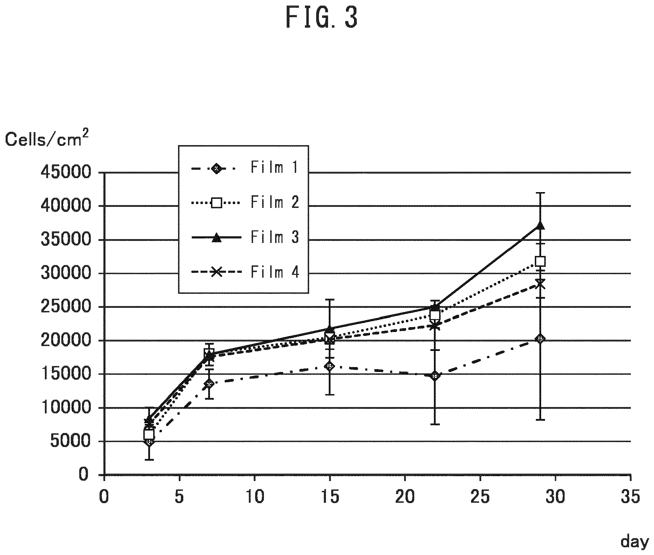

Into a sterilized square container with a size of 2 cm $\times$ 2 cm (Thermo Fisher Scientific Inc., cat. 103), 0.5 mL of a mesenchymal stem cell culture medium (MSCBM, produced by LONZA) was added, and the sterilized 1.4 cm-square films 1 to 4 (those prepared in Example 1) were placed with the mesh-structured surface A up, and the human mesenchymal stem cells in a number of $4\times10^4$ were seeded per sheet. Incubation was continued in a $CO_2$ incubator, and the medium was exchanged periodically twice a week. On day 3, 7, 15, 19, 22, and 29 from the initiation of incubation, the cell number was counted using a CCK 8, and the cell growth behavior was observed. The results are depicted in FIG. 3. It was confirmed that substantially the same number of cells could be stably cultured in the films 1 to 4.

Example 4

Long-term culture of human mesenchymal stem cells on a porous polyimide film

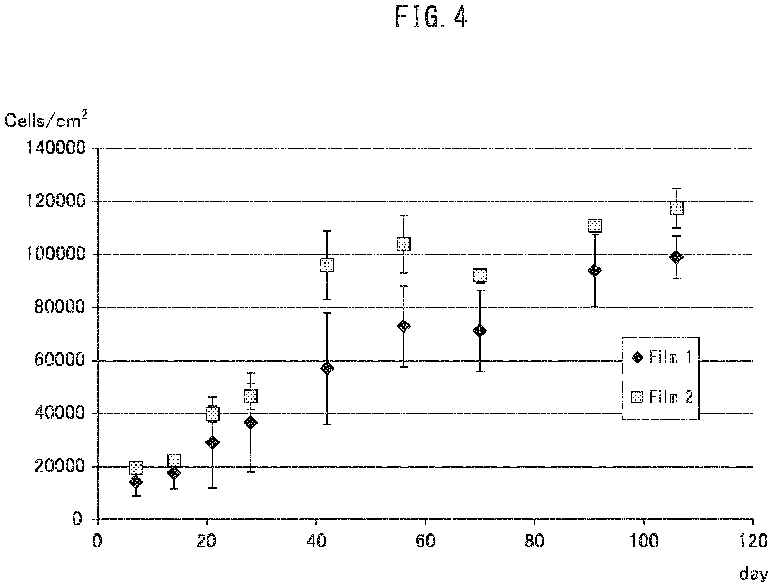

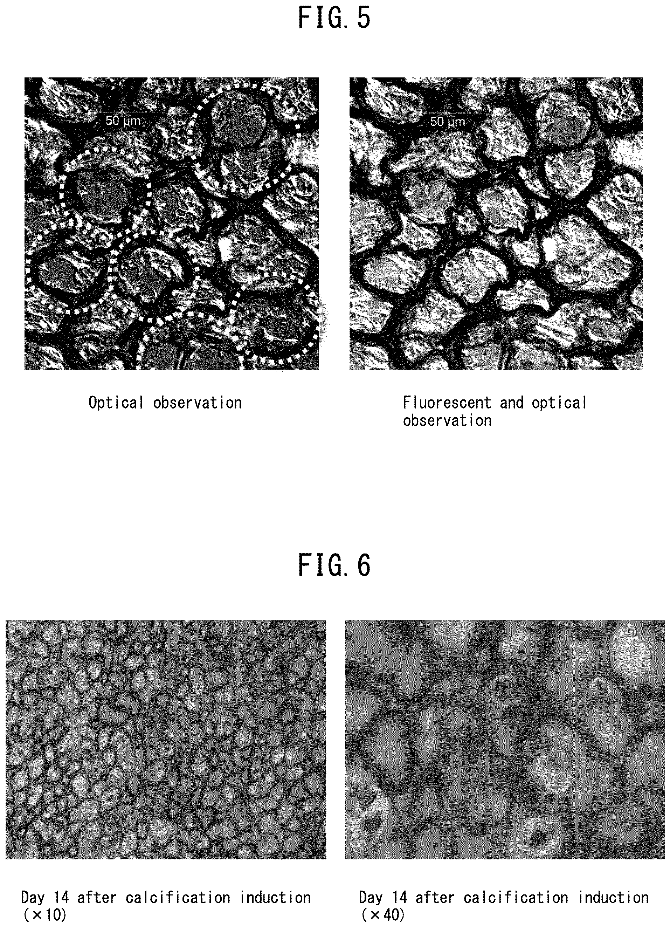

Human mesenchymal stem cells were seeded on a type I collagen coated dish (IWAKI) having a mouth inner diameter of 6 cm, and cultured, and then detached by a trypsin treatment to prepare a cell suspension. Into a sterilized square container with a size of 2 cm $\times$ 2 cm (Thermo Fisher Scientific Inc., cat. 103), 0.5 mL of a cell culture medium (DMEM+FBS 10%, GIBCO) was added, and sterilized 1.4 cm-square porous polyimide films 1 and 2 (those prepared in Example 1) were placed in the container with the mesh-structured surface A up, and the human mesenchymal stem cells in a number of $4\times10^4$ per sheet of porous polyimide film were added to the upper part of the porous polyimide film. Incubation was continued in a $CO_2$ incubator, and the medium was exchanged twice a week. The cell number was counted periodically using a CCK 8, and the cell growth behavior was observed, while continuing the culture. The progress of the cultured cell number up to day 106 is presented in FIG. 4. Stable cell growth was observed. The above-described culture using a porous polyimide film is hereinafter referred to as "member culture" below, and the obtained cell sample is called "member culture cell sample".
The porous polyimide film 2 on day 120 after the initiation of the culture, on which the cells were engrafted, was fixed with formalin, and then stained with Alexa Fluor (registered trademark) 488 phalloidin, CellMask Orange Plasma Membrane Stain, and DAPI. The results of optical observation, and optical and fluorescent observation of the same field with a confocal laser microscope are presented in FIG. 5. The status of cell growth on a yellowish-white porous polyimide film could be optically observed to some extent. High visibility with a yellowish-white porous polyimide film contributes to improvement of observation capability.

Example 5

Induction of differentiation of human mesenchymal stem cells cultured on a porous polyimide film into osteoblasts

The porous polyimide film 1 on day 120 after the initiation of the culture in Example 4, on which the cells were engrafted, was transferred to an osteoblast differentiation-inducing medium (C-28013, produced by PromoCell GmbH), for induction to osteoblasts for 22 days (the medium was exchanged twice a week), and transferred to an osteoblast mineralization medium (C-28020, produced by PromoCell GmbH) for further cultivation for 14 days. Staining was performed with a calcified nodule staining kit (Cosmo Bio Co., Ltd.). The mineralized site was observed with a light microscope. Remarkably reddened sites were recognized to confirm progress of mineralization (FIG. 6). Maintenance of stem cell characteristics of mesenchymal stem cells was confirmed.

Example 6

Induction of differentiation of human mesenchymal stem cells cultured on a porous polyimide film into adipocytes

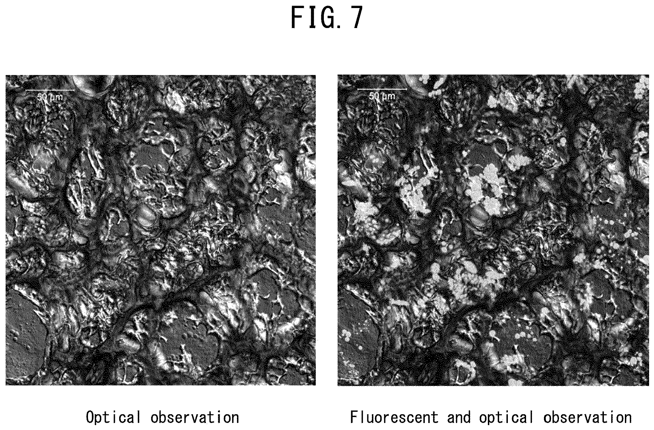

The porous polyimide film 2 on day 127 after the initiation of the culture in Example 4, on which the cells were engrafted, was transferred to an adipocyte inducing medium (C-28016, produced by PromoCell GmbH) for culture for 15 days. The porous polyimide film, on which the cells were engrafted, was fixed in formalin, and oil droplets of adipocytes were fluorescently stained with BODIPY. The results of optical observation, and optical and fluorescent observation of the same field with a confocal laser microscope are presented in FIG. 7. Owing to high visibility of a yellowish-white porous polyimide film, existence of oil droplets was observed not only by fluorescent staining but also by optical observation. Maintenance of stem cell characteristics of mesenchymal stem cell was confirmed.

Example 7

Gene analysis of human mesenchymal stem cells cultured for a long time on a porous polyimide film

1. Preparation of sample

A member culture cell sample with the film 1 on day 154 after the initiation of culture of Example 4 was used as a sample for gene analysis. In addition, human mesenchymal stem cells were cultured for 7 days on a type I collagen-coated dish (IWAKI) under the same conditions as in Example 4 except that a porous polyimide film was not used. The cultured cells were used as a sample for gene analysis. The above culture not using a porous polyimide film is hereinafter referred to as "normal culture", and the obtained cell sample is referred to as a "normal culture cell sample".

2. Gene analysis

Gene analysis was performed on the obtained samples by the following procedure.

(1) RNA extraction

RNA was extracted using an RNeasy Plus Micro Kit (Qiagen) according to the attached protocol. RNA was extracted with 30 $\mu$L of nuclease-free water, and then genomic DNA was digested with DNase using a TURBO DNA-free Kit (Life Technologies). After the digestion treatment, the concentration of the RNA solution was measured with Nano Drop 2000 (Thermo Fisher Scientific).

(2) cDNA synthesis

The RNA solution after the concentration measurement was adjusted to 12.5 ng/$\mu$L, and cDNA synthesis was performed using 100 ng thereof as a template. For synthesis, a SuperScript™ III First-Strand Synthesis System for RT-PCR (Life Technologies) was used. The concentration of the cDNA solution was measured with Nano Drop 2000.

(3) q-PCR reaction

The cDNA solution was adjusted to 200 ng/$\mu$L, and 200 ng thereof was used as a template to perform a measurement by real-time PCR. The PCR was performed with a CFX Connect (Bio-Rad) using SsoAdvanced (trademark) Universal SYBR Green Supermix (Bio-Rad) as a reagent. The expression levels of mesenchymal stem cell positive markers (CD 166, CD 44, CD 105, CD 146, CD 90, CD 106, CD 29, and CD 71), and mesenchymal stem cell negative markers (CD 19, CD 45, CD 31, CD 18, CD 56, CD 34, CD 14, CD 80, CD 40, and CD 86) were measured, in which beta-Actin was used as the inside standard gene.

(4) Analysis of measurement data

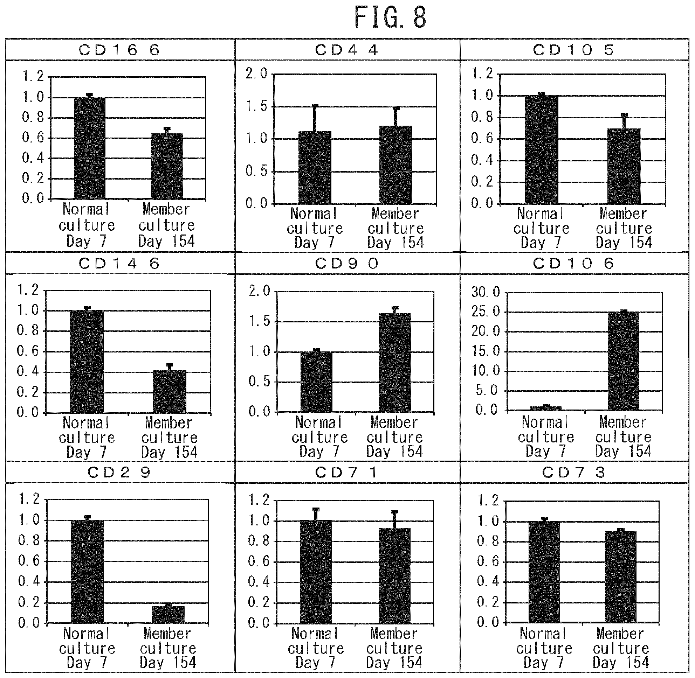

The relative expression levels were calculated from the values obtained by subtracting the measured Ct value of beta-Actin as the inside standard gene from the respective measured Ct values of genes, and were compared each other. Further, in order to compare the time course of gene expression between the normal culture cell sample and the member culture cell sample, the respective changes based on the expression amount of the sample of the normal culture on day 7 as 1 were calculated and compared. The results with respect to the positive markers are presented in FIG. 8. In this regard, it was confirmed that the expression levels of the negative markers were all low. From the results of the gene expression amounts, it was confirmed that the stem cell characteristics were maintained even after a prolonged culture using the film prepared in Example 1.

Example 8

Long-term culture of human dermal fibroblasts using a porous polyimide film

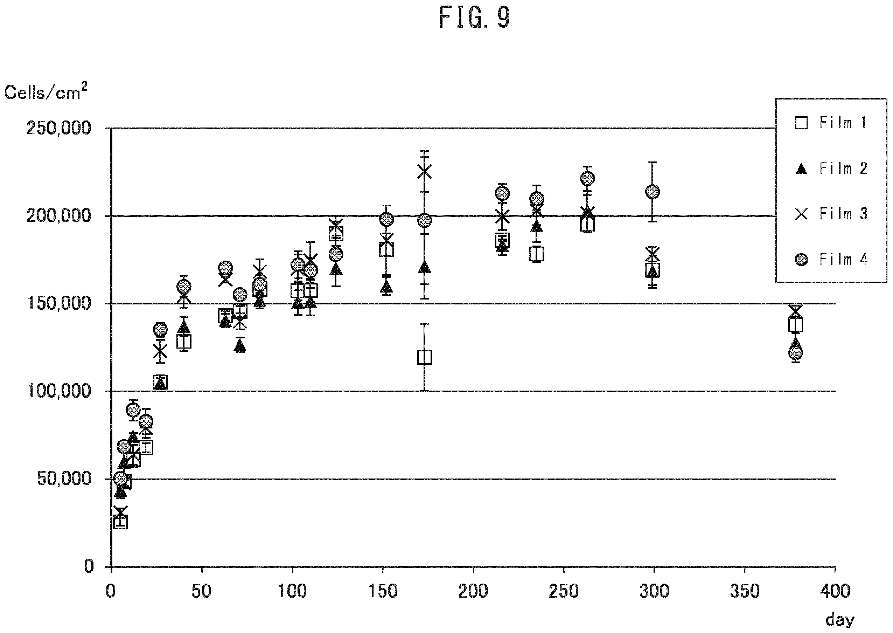

The experiment conducted in Example 1 was further continued, and a long-term culture of about 1 year was carried out. The medium was exchanged twice a week in succession, and the growing cell number was measured appropriately using CCK 8. The results are presented in FIG. 9. Stable proliferation and growth of human dermal fibroblasts were observed even when the culture was continued for a long period of time.

Example 9

Substances produced from human dermal fibroblasts cultured with a porous polyimide film

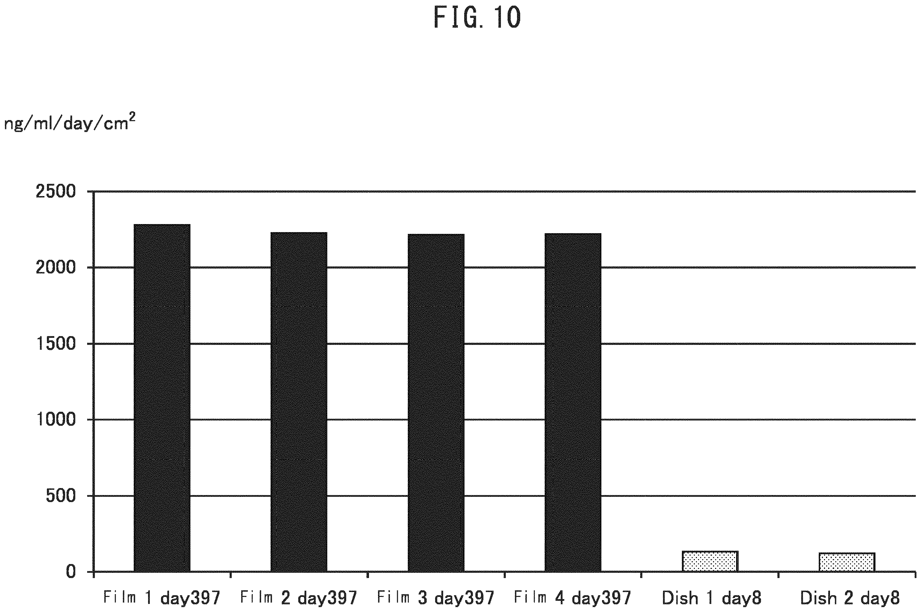

Fibronectin produced from a member culture cell sample on day 397 after the initiation of the culture in Example 8 was measured using an ELISA kit for a human fibronectin assay (Cat# MK115, produced by Takara Bio Inc.,). The results are presented in FIG. 10. Incidentally, using the human dermal fibroblasts cultured for 8 days in cell culture dishes (manufactured by Sumitomo Bakelite Co., Ltd.) under the same conditions as in Example 8 except that a porous polyimide film was not used, fibronectin produced from the cells as a control sample was measured (Dishes 1 and 2 in the figure). Stable fibronectin production was confirmed even after long-term culture, and it was confirmed that the characteristics of the human dermal fibroblast were maintained. It was also confirmed that the efficiency of substance production from the cells cultured with a porous polyimide film is very much higher compared to the efficiency of substance production from the cells cultured in normal culture.

INDUSTRIAL APPLICABILITY

The method of the present invention may be suitably used for simple, efficient, and stable culture of cells. In particular, it is useful because it is possible to visually confirm seeding of cells, the engraftment behavior, and the like. Further, since the porous polyimide film used is colored only slightly, it is advantageously superior in designability.

**Claims**

1. A method for preparing cells, the method comprising the step of: applying cells to a porous polyimide film and culturing the cells;

   wherein the porous polyimide film is a three-layer structure porous polyimide film having a surface layer A and a surface layer B, the surface layers having a plurality of pores, and a macrovoid layer sandwiched between the surface layers A and B;
   wherein an average pore diameter of the pores present in the surface layer A is smaller than an average pore diameter of the pores present in the surface layer B;
   wherein the macrovoid layer has a partition wall bonded to the surface layers A and B, and a plurality of macrovoids surrounded by the partition wall and the surface layers A and B;
   wherein the pores in the surface layers A and B communicate with the macrovoids; and
   wherein the polyimide porous is produced by a method comprising the steps of:

      (1) casting a poly(amic acid) solution consisting of a poly(amic acid) and an organic polar solvent into a film-like shape, and dipping in or bringing it into contact with a coagulating solvent to prepare a porous film of poly(amic acid); and
      (2) imidizing the porous film of the poly(amic acid) obtained in the step (1) by heat treatment.

2. The method for preparing cells according to claim 1, wherein the porous polyimide film is produced by a method comprising the steps of:

   (1) casting a poly(amic acid) solution consisting of a poly(amic acid) and an organic polar solvent into a film-like shape, and dipping in or bringing it into contact with a coagulating solvent to prepare a porous film of poly(amic acid);

(2) imidizing the porous film of the poly(amic acid) obtained in the step (1) by heat treatment; and

(3) subjecting at least one surface of the porous polyimide film obtained in the step (2) to plasma treatment.

3. The method for preparing a cell according to claim 1 or 2, wherein the poly(amic acid) comprises at least one tetracarboxylic dianhydride selected from the group consisting of biphenyltetracarboxylic dianhydride and pyromellitic dianhydride; and at least one diamine selected from the group consisting of benzenediamine, diaminodiphenyl ether and bis(aminophenoxy)phenyl.

4. The method according to any one of claims 1 to 3, the method comprising the step of: seeding cells on the surface of the porous polyimide film.

5. The method according to any one of claims 1 to 3, the method comprising the steps of:

placing a cell suspension on the dried surface of the porous polyimide film;
allowing the porous polyimide film to stand, or moving the porous polyimide film to promote efflux of liquid, or stimulating a part of the surface to cause absorption of the cell suspension into the film; and
retaining cells in the cell suspension in the porous polyimide film, and allowing water to flow out.

6. The method according to any one of claims 1 to 3, the method comprising the steps of:

wetting one or both sides of the porous polyimide film with a cell culture medium or a sterilized liquid;
loading a cell suspension into the wetted porous polyimide film; and
retaining cells in the cell suspension inside the film, and allowing water to flow out.

7. The method according to claim 6, wherein living cells remain within the porous polyimide film, and dead cells flows out with the water.

8. The method according to claim 6 or 7, wherein the sterilized liquid is a sterile water or a sterilized buffer solution.

9. The method according to any one of claims 1 to 8, the method comprising the step of:
placing a cell culture medium, cells, and one or more of the porous polyimide films in a cell culture vessel, wherein the porous polyimide films are in a suspended state in the cell culture medium.

10. The method according to claim 9, **characterized in that** two or more pieces of the porous polyimide films are used.

11. The method according to claim 9 or 10, wherein the cells spontaneously adhere to the porous polyimide film.

12. The method according to any one of claims 1 to 8, wherein the porous polyimide film is

i) folded,
ii) wound into a roll-like shape,
iii) connected as sheets or pieces with a filamentous structure, or
iv) bound into a rope-like shape,
and suspended or fixed in a cell culture medium in a cell culture vessel.

13. The method according to claim 12, wherein cells spontaneously adhere to the porous polyimide film.

14. The method according to any one of claims 1 to 3, the method comprising using two or more porous polyimide films are layered either above and below or left and right in the cell culture medium.

15. The method according to any one of claims 1 to 3, wherein two or more of the methods according to any one of claims 4 to 14 are conducted in combination.

16. The method according to any one of claims 1 to 15, wherein cells grow and proliferate on the surface and the inside of a porous polyimide film.

17. The method according to any one of claims 1 to 16, wherein the cells are selected from the group consisting of animal cells, insect cells, plant cells, yeasts and bacteria.

18. The method according to claim 17, wherein the animal cells are cells derived from an animal belonging to the vertebrate phylum.

19. The method according to claim 17, wherein the bacteria are selected from the group consisting of lactic acid bacteria, Escherichia coli, Bacillus subtilis and cyanobacteria.

20. The method according to any one of claims 1 to 16, wherein the cells are selected from the group consisting of pluripotent stem cells, tissue stem cells, somatic cells and germ cells.

21. The method according to any one of claims 1 to 16, wherein the cells are selected from the group consisting of sarcoma cells, established cells and transformed cells.

22. A cell culture apparatus for use in a method for preparing cells according to any one of claims 1 to 21, the apparatus comprising a porous polyimide film.

23. The cell culture apparatus according to claim 22, wherein two or more porous polyimide films are layered either above and below or left and right.

24. A kit for use in a method for preparing cells according to any one of claims 1 to 21, the kit comprising a porous polyimide film.

## FIG. 1

## FIG. 2

FIG. 3

FIG. 4

# FIG. 5

Optical observation

Fluorescent and optical observation

# FIG. 6

Day 14 after calcification induction (×10)

Day 14 after calcification induction (×40)

# FIG. 7

Optical observation

Fluorescent and optical observation

FIG. 8

EP 3 489 343 A1

# FIG. 9

EP 3 489 343 A1

FIG. 10

EP 3 489 343 A1

# INTERNATIONAL SEARCH REPORT

| International application No. |
| --- |
| PCT/JP2017/026946 |

**A. CLASSIFICATION OF SUBJECT MATTER**

*C12M1/00*(2006.01)i, *B29C41/12*(2006.01)i, *B32B3/12*(2006.01)i, *B32B5/32*(2006.01)i, *B32B27/34*(2006.01)i, *C08J9/28*(2006.01)i, *C12M3/00*(2006.01)i, *C12M3/04*(2006.01)i, *C12N1/00*(2006.01)i, *C12N5/00*(2006.01)i,

According to International Patent Classification (IPC) or to both national classification and IPC

**B. FIELDS SEARCHED**

Minimum documentation searched (classification system followed by classification symbols)

C12M1/00, B29C41/12, B32B3/12, B32B5/32, B32B27/34, C08J9/28, C12M3/00, C12M3/04, C12N1/00, C12N5/00, C12N5/04, C12N5/07, C12N5/10

Documentation searched other than minimum documentation to the extent that such documents are included in the fields searched

Jitsuyo Shinan Koho          1922–1996   Jitsuyo Shinan Toroku Koho   1996–2017
Kokai Jitsuyo Shinan Koho    1971–2017   Toroku Jitsuyo Shinan Koho   1994–2017

Electronic data base consulted during the international search (name of data base and, where practicable, search terms used)

CAplus/MEDLINE/EMBASE/BIOSIS/WPIDS/WPIX(STN)

**C. DOCUMENTS CONSIDERED TO BE RELEVANT**

| Category* | Citation of document, with indication, where appropriate, of the relevant passages | Relevant to claim No. |
| --- | --- | --- |
| Y | WO 2015/012415 A1  (Ube Industries, Ltd.),<br>29 January 2015 (29.01.2015),<br>paragraphs [0072] to [0104]; examples 1 to 12;<br>claims 1 to 23<br>& US 2016/0168560 A1<br>paragraphs [0113] to [0148]; examples 1 to 12;<br>claims 1 to 23<br>& EP 3026108 A1          & CN 105452440 A<br>& KR 10-2016-0034303 A | 1-24 |
| Y | WO 2011/125988 A1  (Ube Industries, Ltd.),<br>13 October 2011 (13.10.2011),<br>paragraphs [0032] to [0059]<br>& US 2013/0045355 A1<br>paragraphs [0078] to [0119]<br>& EP 2557110 A1          & CN 102844362 A<br>& KR 10-2013-0083377 A | 1-24 |

| ☒ Further documents are listed in the continuation of Box C. | ☐ See patent family annex. |
| --- | --- |

| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 28 September 2017 (28.09.17) | 17 October 2017 (17.10.17) |

| Name and mailing address of the ISA/<br>Japan Patent Office<br>3-4-3,Kasumigaseki,Chiyoda-ku,<br>Tokyo 100-8915,Japan | Authorized officer<br><br>Telephone No. |
| --- | --- |

Form PCT/ISA/210 (second sheet) (January 2015)

**EP 3 489 343 A1**

<table>
<tr><td colspan="2" align="center">**INTERNATIONAL SEARCH REPORT**</td><td colspan="2">International application No.<br>PCT/JP2017/026946</td></tr>
</table>

C (Continuation).   DOCUMENTS CONSIDERED TO BE RELEVANT

| Category* | Citation of document, with indication, where appropriate, of the relevant passages | Relevant to claim No. |
|---|---|---|
| Y | WO 2010/038873 A1  (Ube Industries, Ltd.),<br>08 April 2010 (08.04.2010),<br>paragraphs [0032] to [0057]<br>& US 2011/0318556 A1<br>paragraphs [0069] to [0100]<br>& EP 2354180 A1      & CN 102203174 A<br>& KR 10-2011-0079633 A | 1-24 |
| Y | JP 2014-180787 A  (Ube Industries, Ltd.),<br>29 September 2014 (29.09.2014),<br>paragraphs [0017] to [0057]<br>(Family: none) | 1-24 |
| Y | JP 2011-219585 A  (Ube Industries, Ltd.),<br>04 November 2011 (04.11.2011),<br>paragraphs [0034] to [0059]<br>(Family: none) | 1-24 |
| Y | JP 2011-219586 A  (Ube Industries, Ltd.),<br>04 November 2011 (04.11.2011),<br>paragraphs [0032] to [0057]<br>(Family: none) | 1-24 |
| Y | WO 2011/043467 A1  (Ube Industries, Ltd.),<br>14 April 2011 (14.04.2011),<br>paragraphs [0043] to [0060]; table 3,<br>comparative examples 201, 202<br>& US 2012/0207999 A1    & EP 2487196 A1<br>& CN 102575028 A         & KR 10-2012-0093856 A | 1-24 |
| A | IIMARINEN Tanja, et al., "Ultrathin Polyimide<br>Membrane as Cell Carrier for Subretinal<br>Transplantation of Human Embryonic Stem Cell<br>Derived Retinal Pigment Epithelium", PLOS ONE,<br>2015, 10(11), e0143669 | 1-24 |

Form PCT/ISA/210 (continuation of second sheet) (January 2015)

30

| **INTERNATIONAL SEARCH REPORT** | International application No. |
|---|---|
| | PCT/JP2017/026946 |

Continuation of A. CLASSIFICATION OF SUBJECT MATTER
(International Patent Classification (IPC))

C12N5/04(2006.01)i, C12N5/07(2010.01)i, C12N5/10(2006.01)i

(According to International Patent Classification (IPC) or to both national classification and IPC)

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015012415 A
- WO 2009123349 A
- JP 2009057041 W